# EUROPÄISCHE PATENTSCHRIFT

(11) **EP 2 218 473 B1**
(45) Veröffentlichungstag und Bekanntmachung des Hinweises auf die Patenterteilung: **17.08.2011**
(21) Anmeldenummer: 09405028.3
(22) Anmeldetag: 11.02.2009
(51) Int. Cl.: A61M 5/24, A61M 5/28, A61M 5/48, A61M 5/31, A61M 5/315

(54) **Injektionsvorrichtung mit Druckdämpfung für die Injektion eines Fluids**
Injection device with pressure dampening for the injection of a fluid
Dispositif d'injection avec atténuation de pression pour l'injection d'un fluide

(43) Veröffentlichungstag der Anmeldung: 18.08.2010
(73) Patentinhaber: Leuzinger, Nils, 8808 Pfäffikon (CH)
(72) Erfinder: Leuzinger, Nils, 8808 Pfäffikon (CH)
(74) Vertreter: Rentsch Partner AG

(56) Entgegenhaltungen:
- DE-A1- 4 428 467
- DE-A1- 19 503 230
- US-A- 2 655 919
- US-A- 4 064 879
- US-A- 4 403 988
- US-A- 4 710 172
- US-A- 5 314 415
- US-A1- 2003 105 433
- US-A1- 2003 187 406

## Beschreibung

### FELD DER ERFINDUNG

Die vorliegende Erfindung bezieht sich auf das Gebiet Injektionstechnik und insbesondere der medizinischen Injektionstechnik; sie betrifft eine Spritze oder eine Ampulle für eine Karpulenspritze gemäss dem Oberbegriff des Anspruchs 1.

### HINTERGRUND DER ERFINDUNG

Injektionsvorrichtungen unterscheiden sich grundsätzlich in zwei Arten: Zum eine gibt es "gewöhnliche" Aufziehspritzen, umfassend einen mit einem Medikament befüllten Spritzenzylinder, einen handhabbaren Spritzenstempel und eine Kanüle, mit der das Medikament durch Einstechen injiziert werden kann. Zum anderen gibt es so genannte Karpulenspritzen; diese werden nicht wie eine Aufziehspritzen "aufgezogen", sondern durch Einschieben einer Zylinderampulle bestückt. Solche Zylinderampullen bestehen häufig aus einem Glaszylinder, der bereits bei einem Pharmahersteller steril mit einem Medikament abgefüllt werden. An ihrem vorderen Ende ist eine Zylinderampulle -im Folgenden auch kurz "Ampulle" genannt- mit einer Durchstichmembran (Gummi oder Kunststoff) verschlossen, die zur Injektion nach Art eines Injektionsfläschchens von einer Injektionsnadel durchstochen wird. Das hintere Ende der Zylinderampulle ist durch einen Gummistopfen verschlossen, der sich durch den Druck auf einen Stempel der Karpulenspritze wie ein Kolben in den Zylinder einpressen lässt.

Für Karpulenspritzen werden besondere Kanülen (Karpulenkanülen) benötigt. Sie ähneln auf der langen Seite einer ganz normalen Kanüle (angeschliffenes Hohlrohr), mit der das Medikament bzw. ein Lokalanästhetikum gespritzt wird, und haben am anderen Ende ein weiteres kurzes angeschliffenes Hohlrohr, mit dem die Durchstichmembran der Zylinderampulle durchstochen wird. Die Karpulenkanülen werden auf die Karpulenspritzen aufgeschraubt.

Karpulenspritzen finden bekanntermassen Anwendung in der Medizin für die Lokalanästhesie, und im speziellen in der Dentalmedizin, da hier die Lokalanästhesie eine der häufigsten Vorbehandlungen darstellt. Lokalanästhetika werden meistens mit einer Injektionsspritze in das zu behandelnde Gewebe appliziert. Dies führt trotz der heutigen Applikationstechniken -die eine möglichst schmerzarme Anästhesie gewährleisten sollen- zu unangenehmen Schmerzen bei Patienten. Diese Schmerzen werden hauptsächlich durch zwei Quellen verursacht: Einerseits durch den Einstich einer Applikationsnadel und andererseits durch die Ausdehnung des injizierten Lokalanästhetikums ins Gewebe (Gewebedehnung). Dabei wird vor allem die Ausdehnung des Gewebes als schmerzhaft empfunden, insbesondere wenn diese Ausdehnung schnell und dynamisch bzw. ruckartig erfolgt. Darüber hinaus können mit zu hohem Druck applizierte Substanzen sogar zu schmerzhaften Nekrosen (Absterben des Gewebes) führen.

Durch die verschiedenartige Beschaffenheit des Gewebes, ist das lokale Schmerzempfinden an verschiedenen Körperstellen unterschiedlich. Insbesondere wenig dehnbare Gewebe wie das Palatum durum (Gaumen) sind sehr schmerzempfindlich, wenn die Injektion zu schnell und ruckartig erfolgt. Gut dehnbare Gewebe wie das Vestibulum oris (Mundvorhof) sind hingegen weniger schmerzempfindlich.

Grundsätzlich existieren zwei Gruppen von Spritzenanordnungen in Bezug auf ihre Antriebsart, namentlich handbetriebene und solche mit einem elektrischen Antrieb. Bei handbetriebenen Spritzenanordnungen spielt die Feinmotorik -beispielsweise eines Zahnrzts- eine wesentliche Rolle. Ein Zahnarzt ist zwar sehr schnell in der Lage, Schmerzreaktionen eines Patienten beim Spritzen wahrzunehmen, hingegen bedarf es eines hohen Masses an feinmotorischen Fähigkeiten, um die Injektionsgeschwindigkeit möglichst rasch anzupassen und konstant zu halten bzw. die Injektionsdynamik entsprechend klein zu halten. Die Bewegung eines Daumens ist feinmotorisch allerdings schwierig steuerbar und neigt zu ruckartigen Bewegungen. Diese ruckartigen Bewegungen des Daumens werden direkt auf den Kolben einer Injektionsspritze übertragen und führen somit zu unregelmässigen Druckspitzen in der Injektionsflüssigkeit und entsprechenden Schmerzsensationen im Gewebe. Auf der anderen Seite zeigen Spritzen mit elektrischer oder anders gearteter Antriebstechnik eine äusserst konstante Injektionsgeschwindigkeit, sie sind aber nicht in der Lage, das grundsätzlich subjektiv unterschiedlich wahrgenommene Schmerzempfinden eines Patient während der Injektion zu berücksichtigen.

Zudem ist beispielsweise bei einer Lokalanästhesie des Unterkiefers eine Aspiration obligat - nach dem Einstich bis in die endgültige Position wird leicht angesaugt, um zu kontrollieren, ob die Kanüle nicht in einem Blutgefäß liegt, was unbedingt vermieden werden soll. Eine solche Aspirationsprobe ist bei konventionellen Spritzen mit Handbetrieb mittels Zug an einem Spritzenstempel vergleichsweise einfach durchführbar; bei Spritzen mit einem eigenen Antrieb stellt die Aspirationsprobe eine zusätzlich Herausforderung dar.

Konstruktiv ist das Problem bei der Karpulenspritze dadurch gelöst, dass durch Drehung an einem Spritzenstempel und eine innere Achse im Spritzenstempel drei Häkchen ausfahren (Aspirationshaken), die sich in den Gummistopfen der Zylinderampulle krallen. Der Gummistopfen der Zylinderampulle hat zu diesem Zweck auf der unsterilen Rückseite ein zylinderförmiges Loch.

Eine andere, etwas einfachere konstruktive Lösung besteht aus einem Widerhaken. Hierbei gibt es keine beweglichen Teile (innere Achse, drei bewegliche Häkchen), sondern an Stelle der drei beweglichen Häkchen ist ein Widerhaken angebracht (Harpunenform oder flache viereckige Platte), der sich in das zylinderförmige Loch des Gummistopfens krallt.

Durch diese recht provisorische Verbindung zwischen Spritzenstempel und Zylinderampullen-Stopfen kann zwecks Aspirationsprobe ein leichter Unterdruck in der Zylinderampulle aufgebaut werden.

Da der Zahnarzt in der anderen Hand meist einen Spiegel zum Abhalten der Wange oder Lippe hält, muss die Aspirationsprobe einhändig durchgeführt werden können. Deshalb sind neuere Modelle der Karpulenspritzen meist mit einem Daumenring versehen.

Auch Karpulenspritzen mit eigenem Antrieb stellen solche beschriebenen Verbindungen zwischen Spritzenstempel und Zylinderampulle bereit; darüber hinaus muss der Antrieb in beiden Richtungen, namentlichen Druck (Injektion) und Vakuum (Aspiration) funktionieren, was eine Umschaltung zwischen beiden Antriebsrichtungen erforderlich macht.

Aus der DE 601 19 354 T2 ist eine Dentalanästhesie-Aufziehpritze bekannt, bei der ein Spritzenhandgerät mit einem Kontrollgerät verbunden ist und das Spritzenhandgerät einen Schrittmotor mit Schraubenspindelantrieb aufweist. Das Kontrollgerät ist in der Lage, ein Anästhetikum mit einer ersten Injektionsrate (Milliliter/Zeiteinheit) für eine erste Injektionsdauer zuzuführen und zudem mit einer zweiten Injektionsrate über eine zweite Injektionsdauer. Eine weitere Anpassung an die doch sehr individuelle Schmerzempfindlichkeit von Patienten ist nicht möglich.

Die aus der WO 03/004081 A1 bekannte, "gewöhnliche" Spritzenanordnung weist einen elastischen Kolben auf, der bei Injektion aufgrund des Widerstands gegenüber einem Medikament deformierbar ist, um eine Dämpfungswirkung beim Injizieren zu erreichen. Als nachteilig kann hier die Trägheit dieser Dämpfungswirkung mittels verformbarem Kolben angesehen werden.

Eine Drucküberwachungsvorrichtung für eine Aufziehspritzenanordnung ist aus der WO 93/01573 bekannt, welche Vorrichtung am handbedienten Ende eines Spritzenstempels aufsetzbar ist. Diese Drucküberwachungsvorrichtung ist zwar sehr flexibel einsetzbar -das heisst wieder verwendbar an verschiedenen Spritzen- hingegen bedarf es dennoch eines ausgeprägten feinmotorischen Gespürs für die konstante Federkraft der Dämpfungselements, um möglichst schmerzfrei Behandlungen durchführen zu können.

Die DE 195 03 230 A1 stellt eine Spritze mit einer in den Kolben integrierten Dämpfungs- und Druckmesseinrichtung und Verfahren zur vergleichmässigten und druckkontrollierten Injektion mittels dieser Spritze dar. Hier dient das Anästhetikum gleichzeitig abströmseitig als Medikament und innerhalb des Spritzenkolbens als barometrisches Anzeigefluid, das abhängig vom Druck, der auf den Spritzenstempel ausgeübt wird in eine Kapillare getrieben wird. Abgesehen davon, dass diese Spritzenvorrichtung gegenüber anderen bekannten Ausführungsformen aufwendig gestaltet ist, kann durch Betrachten der Druckanzeige beim Injizieren zwar eine Aussage über den Druck gemacht werden, hingegen sollte das nicht das Entscheidungskriterium für die Injektionsgeschwindigkeit sein, sondern rein die individuelle Schmerzreaktion eines Patienten.

In der US 2003/0105433 A1 und der DE 44 28 467 A1 ist jeweils eine Injektionsvorrichtung zum Injizieren eines Fluids offenbart, umfassend eine Kanüle, einen Spritzenstempel und einen mit diesem Fluid befüllten Spritzenzylinder einer Aufziehspritze oder einer mit diesem Fluid befüllten Ampulle für eine Karpulenspritze wobei in dem Spritzenzylinder oder der Ampulle zur Kanüle hin das Fluid angeordnet ist und auf der der Kanüle abgewandten Seite des Fluids ein erster Kolben den Spritzenzylinder oder die Ampulle verschliesst und ein zweiter Kolben so an einem der Kanüle abgewandten Ende des Spritzenzylinders oder der Ampulle angeordnet ist, dass zwischen dem ersten und dem zweiten Kolben ein Gasvolumen als Dämpfungselement dicht eingeschlossen ist und der zweite Kolben mittels des Spritzenstempels bedienbar ist.

### DARSTELLUNG DER ERFINDUNG

Es ist daher Aufgabe der Erfindung, eine Injektionsvorrichtung für die Injektion eines Fluid zu schaffen, mit der möglichst scherzarm ein Medikament injiziert werden kann, wobei diese Injektionsvorrichtung einfach aufgebaut ist und in einfacher bekannter Weise anwendbar ist.

Die der Erfindung zugrunde liegende Aufgabe wird durch die Gesamtheit der Merkmale des Anspruchs 1 gelöst. Weitere Ausführungsformen sind Gegenstand der abhängigen Ansprüche 2 bis 7.

Für die Erfindung wesentlich ist, dass die Injektionsvorrichtung mit zwei Kolben versehen ist, wobei zwischen beiden Kolben ein Gasvolumen als Dämpfungselement eingeschlossen ist, und wobei innerhalb des Gasvolumens an dem ersten und dem zweiten Kolben eine Druckanzeigevorrichtung angeordnet ist. Wenn der aussen liegende Kolben mittels eines Spritzenstempels betätigt wird, dann wird zunächst das Gasvolumen komprimiert und der innen liegende Kolben durch das komprimierte Gasvolumen gedämpft angetrieben. Bei einer Bedienung dieser neuen Injektionsvorrichtung ist somit gegenüber dem Stand der Technik eine vergleichsweise feinere und gleichmässigere Dosierung eines Fluids möglich und Druckspitzen können weitgehend geglättet werden. Folglich können Ausdehnungsschmerzen in einem Gewebe wesentlich reduziert werden.

Die Druckanzeigevorrichtung umfasst ein erstes Anzeigeelement an dem ersten Kolben und ein zweites Anzeigeelement an dem zweiten Kolben und zwar derart, dass sich die beiden Anzeigeelemente bei Betätigung der Injektionsanordnung zwecks Druckanzeige aufeinander zubewegen.

Erfindungsgemäß das erste Anzeigeelement ein Stift und das zweite Anzeigeelement eine Hülse, wobei der Stift so angeordnet ist, dass er von der Hülse führend umfasst ist. Vorzugsweise ist der Stift mit einer Markierung oder einer Anzahl Markierungen versehen, sodass anhand dieser Markierungen eine analoge Anzeige des ausgeübten Drucks auf den Spritzenstempels beim Aufeinanderzubewegen erkennbar ist. Solche Markierungen können eine Skalierung mit feinen Strichen oder Ringen umfassen oder eine entlang des Stifts angeordnete Nummerierung oder eine oder mehrere Einfärbungen.

Eine bevorzugte Ausgestaltung der Erfindung sieht vor, dass die Hülse zumindest teilweise transparent ausgebildet ist und an ihrem freien Ende einen opaken Ring aufweist. Wenn sich die Kolben im Betrieb aufeinander zubewegen, wird der in der Hülse geführte Stift im transparenten Bereich der Hülse sichtbar. Im Ruhezustand der beiden Kolben ist ein freies Stiftende des Stifts von dem opaken Ring verdeckt.

Mit Vorteil ist bei einer Aufziehspritze der zweite -aussen liegende-Kolben und der Spritzenstempel einstückig ausgeführt.

In einem nicht erfindungsgemäßen Beispiel ist es weiters möglich, dass das Gasvolumen als ein Rückstossvolumen für eine Aspirationsprobe fungiert. Hierfür ist im Ruhezustand beider Kolben ein leichter Unterdruck im Gasvolumen vorgesehen, der klein genug ist, dass sich aufgrund der Haftreibung der Kolben mit einer Innenwand der Injektionsvorrichtung die Kolben ohne Betätigung nicht aufeinander zubewegen. Erst wenn eine Kanüle ins Gewebe eingeführt wird und gegebenenfalls eine Ader getroffen wird ergänzen sich er Unterdruck im Gasvolumen mit dem Druck in der Ader so, dass sich die Kolben selbständig aufeinander zubewegen. Auf diese Art lassen sich Aspirationsproben ohne jede weitere Betätigung eines Spritzenstempels durchführen.

### KURZBESCHREIBUNG DER FIGUREN

Die Erfindung soll nachfolgend anhand von Ausführungsbeispielen im Zusammenhang mit der Zeichnung näher erläutert werden. Es zeigen
- Fig. 1: eine vereinfachte, schnittbildliche Darstellung einer Injektionsvorrichtung in Form einer Ampulle für eine Karpulenspritze nach dem Stand der Technik;
- Fig. 2: eine Karpulenspritze mit einer solchen Ampulle gemäss Fig. 1 nach dem Stand der Technik;
- Fig. 3: eine Injektionsvorrichtung in Form einer Aufziehspritze nach dem Stand der Technik;
- Fig. 4a: eine Ausführungsform der Injektionsvorrichtung mit einer Druckanzeigevorrichtung in Ruheposition, und
- Fig. 4b: eine solche Druckanzeigevorrichtung nach Fig. 4a in einer Betriebsposition.

### DETAILLIERTE BESCHREIBUNG BEVORZUGTER AUSFÜHRUNGSFORMEN

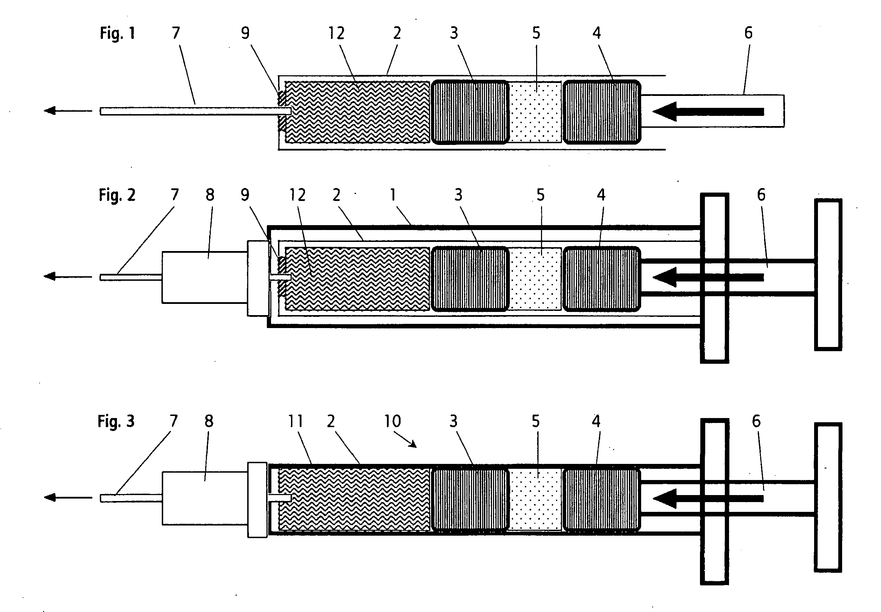

Fig. 1 zeigt den Grundaufbau der Injektionsvorrichtung nach dem Stand der Technik anhand einer Ampulle 2, wie sie typischerweise mit einer Karpulenspritze (10, siehe Fig. 2) zum Einsatz gelangen kann. In der Ampullen 2 sind neben einer Kanüle 7 ein zu injizierendes Fluid 12, ein erster Kolben 3 und eine zweiter Kolben 4 angeordnet, wobei zwischen diesen Kolben 3, 4 ein hermetisch eingeschlossenes Gasvolumen 5 angeordnet ist. Die Kanüle durchsticht an der Fluid-Seite der Ampulle 2 eine Durchstichmembran 9; auf der anderen Seite der Ampulle 2 weist ein Spritzenstempel 6 eine Wirkverbindung mit dem zweiten Kolben 4 auf.

In Fig. 2 ist eine Karpulenspritze 1 gezeigt, und zwar mit einer in sie eingesetzten Ampulle 2 gemäss der Fig. 1. Hierin ist zudem eine schraubbare Kanülenhalterung 8 gezeigt, die die Kanüle 7 umgibt und an der Karpulenspritze 1 sichert.

Eine Aufziehspritze 10 mit einer Injektionsvorrichtung nach dem Stand der Technik ist in Fig. 3 gezeigt. Ein wesentlicher Unterschied zur Karpulenspritze besteht darin, dass die Doppelkolbenanordnung -umfassend den ersten und den zweiten Kolben 3, 4- mit eingeschlossenem Gasvolumen 5 unmittelbar in einem Spritzenzylinder 11 verbaut sind; dieser Spritzenzylinder 11 übernimmt hier die Funktion der Ampulle 2 gemäss den Figuren 1, 2. Zudem kann der Spritzenstempel 6 hier einstückig mit dem zweiten Kolben 4 ausgebildet sein.

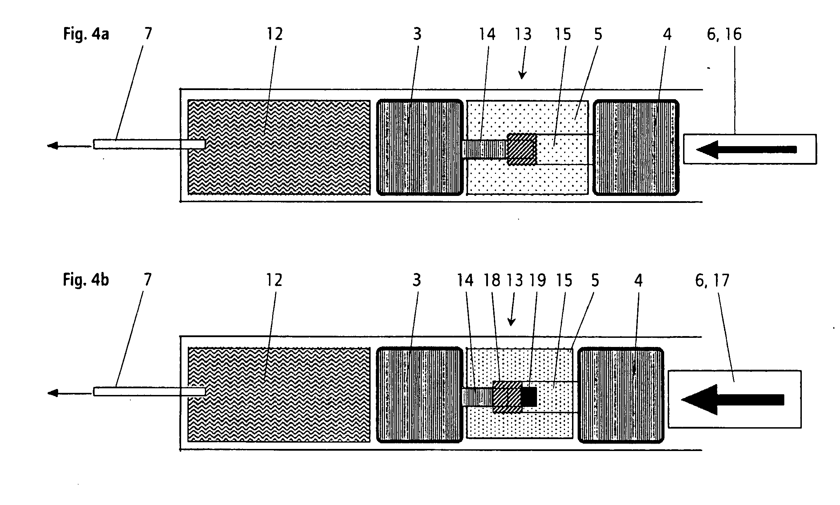

In den Figuren 4a, 4b ist eine Ausführungsform der erfindungsgemässen Injektionsvorrichtung dargestellt; grundsätzlich sind wieder die Kanüle 7, das Fluid 12, der erste Kolben 3 das Gasvolumen 5, der zweite Kolben 4 und der Spritzenstempel 6 in einer Reihenschaltung wirkverbunden. Des Weiteren weist diese Ausführungsform eine Druckanzeigevorrichtung 13 mit einem ersten und einem zweiten Anzeigeelement 14, 15 auf.

Das erste Anzeigeelement 14 ist am ersten Kolben 3 angeordnet und das zweite Anzeigeelement 15 am zweiten Kolben 4. Dabei ist das erste Anzeigeelement 14 als ein Stift ausgebildet, während das zweite Anzeigeelement 15 eine Hülse darstellt, deren Durchmesser leicht grösser ist als der des Stifts, so dass letzterer in der Hülse beweglich gehaltert ist. Wie die Fig. 4b erhellt, ist die im wesentlichen transparente Hülse mit einem opaken Ring 18 am ihrem freien Ende versehen, so dass ein freies Stiftende 19 im transparenten Teil der Hülse sichtbar wird, wenn in einem Betriebszustand das Gasvolumen 5 entsprechend komprimiert ist.

Unter Zuhilfenahme der Fig. 1 - 3 stellt sich die Funktionsweise der neuen Injektionsvorrichtung wie folgt dar: Sobald mittels Betätigung des Spritzenstempels 6 der zweite Kolben 4 zum Gasvolumen 5 hin verschoben wird, komprimiert sich das Gasvolumen 5 bis der Druck so gross wird, dass der erste Kolben 3 nun auch verschoben wird und Fluid 12 aus der Kanüle 7 gefördert wird. Das Gasvolumen 5 wirkt hier als glättendes Dämpfungselement zwischen den beiden Kolben 3, 4, so dass das Fluid 12 ohne jede Druckspitze injizierbar ist und somit erfindungsgemäss die Schmerzsensationen bei Patienten auf ein sehr geringes Mass reduzierbar sind.

Die Druckanzeigevorrichtung 13 ermöglicht es, mittels der beiden Anzeigeelemente 14, 15 auf einfache Art den Druck im Gasvolumen 5 anzuzeigen, wenn der Stift in die Hülse eintaucht in Abhängigkeit von der Grösse des Drucks. Wie die Figuren 4a, 4b erhellen, ist bei niedrigem Druck auf den Spritzenstempel 6 -siehe dünner Druckpfeil 16- im transparenten Teil der Hülse nichts zu sehen, wohingegen bei grossem Druck auf den Spritzenstempel 6 -siehe dicker Druckpfeil 17- das Gasvolumen 5 so komprimiert wird, dass das Ende 19 des Stifts in den sichtbaren .Bereich der Hülse gelangt. Diese Druckanzeige 13 ist sowohl in Ampulle 2 -wie in den Fig. 1, 2 gezeigt- als auch in Aufziehspritzen 10 -wie in Fig. 3 gezeigt- anwendbar.

Wenn im Ruhezustand das Gasvolumen 5 gegenüber der Umgebung einen niedrigeren Druck aufweist, ohne das dadurch die Haftreibung der beiden Kolben 3, 4 überwunden wird, kann mit der erfindungsgemässen Injektionsvorrichtung auf besonders einfache Art eine Aspirationsprobe durchgeführt werden; sobald nämlich mit einer Kanüle 7 eine Ader mit höherem Blutdruck getroffen wird, wirkt dieser Blutdruck mit dem Unterdruck im Gasvolumen 5 derart zusammen, dass quasi eine automatische Aspirationsprobe durchführbar ist, ohne dass an einem Spritzenstempel 6, wie bei den Ausführungsformen des Standes der Technik, mittels entsprechender Handhabung gezogen werden muss. Somit kann bereits eine Aspirationsprobe möglichst schmerzfrei durchgeführt werden, da die Injektionsvorrichtung gemäss der Erfindung ohne jede weitere Manipulation lediglich eingeführt werden muss.

Insgesamt ergibt sich mit der Erfindung eine einfach aufgebaute und leicht zu handhabende Injektionsvorrichtung, mit der beispielsweise eine äusserst schmerzarme Anästhesie möglich ist, wobei vorgängig eine quasi automatische Aspirationsprobe durchführbar ist.

### Bezugszeichenliste

- 1: Karpulenspritze
- 2: Ampulle
- 3: erster Kolben
- 4: zweiter Kolben
- 5: Dämpfungselement, Gasvolumen
- 6: Spritzenstempel
- 7: Kanüle
- 8: Kanülenhalterung, schraubbar
- 9: Durchstichmembran
- 10: Aufziehspritze
- 11: Spritzenzylinder von 10
- 12: Fluid
- 13: Druckanzeigevorrichtung
- 14: erstes Anzeigeelement, Stift
- 15: zweites Anzeigeelement, Hülse
- 16: Druckpfeil, dünn
- 17: Druckpfeil, dick
- 18: Ring an 15
- 19: Stiftende

## Patentansprüche

1. Injektionsvorrichtung zum Injizieren eines Fluids (12), umfassend eine Kanüle (7), einen Spritzenstempel (6) und einen mit diesem Fluid (12) befüllten Spritzenzylinder (11) einer Aufziehspritze (10) oder einer mit diesem Fluid (12) befüllten Ampulle (2) für eine Karpulenspritze (1) wobei in dem Spritzenzylinder (11) oder der Ampulle (2) zur Kanüle (7) hin das Fluid (12) angeordnet ist und auf der der Kanüle (7) abgewandten Seite des Fluids ein erster Kolben (3) den Spritzenzylinder (11) oder die Ampulle (2) verschliesst und ein zweiter Kolben (4) so an einem der Kanüle (7) abgewandten Ende des Spritzenzylinders (11) oder der Ampulle (2) angeordnet ist, dass zwischen dem ersten und dem zweiten Kolben (3, 4) ein Gasvolumen (5) als Dämpfungselement dicht eingeschlossen ist und der zweite Kolben (4) mittels des Spritzenstempels (6) bedienbar ist so dass Druck auf den Spritzenstempel (6) und den zweiten Kolben (4) durch das Gasvolumen (5) gedämpft auf den ersten Kolben (3) und somit auf das Fluid (12) übertragen wird, wobei innerhalb des Gasvolumens (5) an dem ersten und dem zweiten Kolben (3, 4) eine Druckanzeigevorrichtung (13) angeordnet ist und ein erstes Anzeigeelement (14) an dem ersten Kolben (3) und ein zweites Anzeigeelement (15) an dem zweiten Kolben (4) derart angeordnet sind, dass sie zwecks Druckanzeige aufeinander zubewegbar sind, **dadurch gekennzeichnet, dass** das erste Anzeigeelement (14) ein Stift ist und das zweite Anzeigeelement (15) eine Hülse ist, wobei der Stift so angeordnet ist, dass er verschiebbar in die Hülse eingreift.

2. Injektionsvorrichtung nach Anspruch 1, **dadurch gekennzeichnet, dass** der Stift eine Anzahl Markierungen aufweist, zwecks analoger Anzeige einer gegenseitigen Verschiebung zwischen diesem Stift und der Hülse.

3. Injektionsvorrichtung nach Anspruch 2, **dadurch gekennzeichnet, dass** die Markierung eine Skalierung und/oder eine Nummerierung und/oder eine Einfärbung ist.

4. Injektionsvorrichtung nach einem der Ansprüche 2 oder 3, **dadurch gekennzeichnet, dass** die Hülse zumindest teilweise transparent ist, so dass der in ihr geführte Stift darin sichtbar ist.

5. Injektionsvorrichtung nach Anspruch 4, **dadurch gekennzeichnet, dass** die Hülse zu ihrem freien Ende hin einen opaken Ring (18) aufweist, der im Ruhezustand der beiden Kolben (3, 4) ein freies Stiftende (19) des Stifts überdeckt.

6. Injektionsvorrichtung nach einem der Ansprüche 1 bis 5 **dadurch gekennzeichnet, dass** in einer Aufziehspritze (10) der zweite Kolben (4) und der Spritzenstempel (6) einstückig ausgeführt sind.

7. Injektionsvorrichtung nach einem der Ansprüche 1 bis 5, **dadurch gekennzeichnet, dass** die Ampulle (2) auswechselbar in der Karpulenspritze (1) angeordnet ist, wobei die Kanüle (7) an einer Seite der Ampulle (2) durch eine Durchstichmembran (9) einstechbar ist.

## Claims

1. Injection device for the injection of a fluid (12), comprising a needle (7), a syringe plunger (6), and, filled with this fluid (12), a syringe barrel (11) of a pre-filled syringe (10) or, filled with this fluid (12), an ampoule (2) for a carpule syringe (1), wherein the fluid (12) in the syringe barrel (11) or in the ampoule (2) is arranged towards the needle (7), and, on the side of the fluid directed away from the needle (7), a first piston (3) closes off the syringe barrel (11) or the ampoule (2), and a second piston (4) is arranged on an end of the syringe barrel (11) or of the ampoule (2) directed away from the needle (7) in such a way that a gas volume (5) is tightly enclosed as a damping element between the first and second pistons (3, 4), and the second piston (4) can be operated by means of the syringe plunger (6), such that pressure on the syringe plunger (6) and on the second piston (4) is damped by the gas volume (5) and transferred to the first piston (3) and thus to the fluid (12), wherein a pressure display device (13) is arranged on the first and second pistons (3, 4) inside the gas volume (5), and a first display element (14) on the first piston (3) and a second display element (15) on the second piston (4) are arranged in such a way that they can be moved towards each other for the purpose of pressure display, **characterized in that** the first display element (14) is a pin and the second display element (15) is a sleeve, the pin being arranged such that it engages movably in the sleeve.

2. Injection device according to Claim 1, **characterized in that** the pin has a number of markings for the purpose of analogue display of a mutual displacement between this pin and the sleeve.

3. Injection device according to Claim 2, **characterized in that** the marking is a scale and/or numbers and/or colours.

4. Injection device according to either of Claims 2 and 3, **characterized in that** the sleeve is at least partially transparent, such that the pin guided in the sleeve is visible therein.

5. Injection device according to Claim 4, **characterized in that** the sleeve, towards its free end, has an opaque ring (18), which covers a free end (19) of the pin when the two pistons (3, 4) are in the rest state.

6. Injection device according to one of Claims 1 to 5, **characterized in that**, in a pre-filled syringe (10), the second piston (4) and the syringe plunger (6) are designed in one piece.

7. Injection device according to one of Claims 1 to 5, **characterized in that** the ampoule (2) is arranged replaceably in the carpule syringe (1), wherein the needle (7) on one side of the ampoule (2) can be pushed through a pierceable membrane (9).

## Revendications

1. Dispositif d'injection pour l'injection d'un fluide (12), comprenant une aiguille (7), un poussoir de seringue (6) et un corps de seringue (11) rempli de ce fluide (12) d'une seringue d'aspiration (10) ou d'une ampoule (2) remplie de ce fluide (12) pour une seringue à carpule (1), le fluide (12) étant disposé dans le corps de seringue (11) ou dans l'ampoule (2) vers l'aiguille (7), et du côté du fluide opposé à l'aiguille (7), un premier piston (3) fermant le corps de seringue (11) ou l'ampoule (2) et un deuxième piston (4) étant disposé sur une extrémité de l'ampoule (2) ou du corps de seringue (11) opposée à l'aiguille (7) de telle sorte qu'entre le premier et le deuxième piston (3, 4) soit renfermé hermétiquement un volume de gaz (5) en tant qu'élément d'amortissement, et le deuxième piston (4) pouvant être actionné au moyen du poussoir de seringue (6), de sorte que la pression sur le poussoir de seringue (6) et le deuxième piston (4) soit transmise sous forme amortie par le volume de gaz (5) au premier piston (3) et donc au fluide (12), un dispositif d'indication de pression (13) étant disposé à l'intérieur du volume de gaz (5) sur le premier et le deuxième piston (3, 4), et un premier élément d'affichage (14) étant disposé sur le premier piston (3) et un deuxième élément d'affichage (15) étant disposé sur le deuxième piston (4), de telle sorte qu'ils puissent être déplacés l'un vers l'autre en vue d'afficher la pression, **caractérisé en ce que** le premier élément d'affichage (14) est une goupille et le deuxième élément d'affichage (15) est une douille, la goupille étant disposée de telle sorte qu'elle puisse venir en prise de manière déplaçable dans la douille.

2. Dispositif d'injection selon la revendication 1, **caractérisé en ce que** la goupille présente une pluralité de marquages, afin d'afficher de manière analogique un déplacement mutuel entre cette goupille et la douille.

3. Dispositif d'injection selon la revendication 2, **caractérisé en ce que** le marquage est une graduation et/ou une numérotation et/ou un marquage coloré.

4. Dispositif d'injection selon l'une quelconque des revendications 2 ou 3, **caractérisé en ce que** la douille est au moins en partie transparente, de sorte que la goupille guidée à l'intérieur de celle-ci puisse être visible.

5. Dispositif d'injection selon la revendication 4, **caractérisé en ce que** la douille présente, vers son extrémité libre, une bague opaque (18), qui, dans l'état de repos des deux pistons (3, 4), recouvre une extrémité libre (19) de la goupille.

6. Dispositif d'injection selon l'une quelconque des revendications 1 à 5, **caractérisé en ce que** dans une seringue d'aspiration (10), le deuxième piston (4) et le poussoir de seringue (6) sont réalisés d'une seule pièce.

7. Dispositif d'injection selon l'une quelconque des revendications 1 'à 5, **caractérisé en ce que** l'ampoule (2) est disposée de manière remplaçable dans la seringue à carpule (1), l'aiguille (7) pouvant être enfoncée à travers une membrane à percer (9) d'un côté de l'ampoule (2).
